# EUROPEAN PATENT SPECIFICATION

(11) **EP 3 167 289 B1**
(45) Date of publication and mention of the grant of the patent: **11.03.2020**
(21) Application number: 15818893.8
(22) Date of filing: 13.07.2015
(51) Int. Cl.: G01N 33/68

(54) **SRM/MRM ASSAY FOR THE SERINE/THREONINE-PROTEIN KINASE B-RAF (BRAF)**
SRM-/MRM-ASSAY FÜR SERIN-/THREONIN-PROTEINKINASE B-RAF (BRAF)
DOSAGE PAR SRM/MRM DE LA PROTÉINE SÉRINE/THRÉONINE KINASE B-RAF (BRAF)

(30) Priority: 11.07.2014 US 201462023615 P
(43) Date of publication of application: 17.05.2017
(73) Proprietor: Expression Pathology, Inc., Rockville, MD 20850 (US)
(72) Inventor: KRIZMAN, David, B., Gaithersburg, MD 20882 (US); HEMBROUGH, Todd, Gaithersburg, MD 20882 (US); THYPARAMBIL, Sheeno, Frederick, MD 21704 (US); LIAO, Wei-Li, Herndon, VA 20170 (US)
(74) Representative: AWA Sweden AB
(86) International application number: PCT/US2015/040202
(87) International publication number: WO 2016/007959

(56) References cited:
- WO-A1-2014/037977
- CN-B- 101 838 683
- US-A1- 2012 065 140
- US-A1- 2013 068 943
- US-A1- 2013 289 142
- US-A1- 2013 289 142
- US-A1- 2014 011 748
- ROSLYN DILLON ET AL: "Discovery of a Novel B-Raf Fusion Protein Related to c-Met Drug Resistance", JOURNAL OF PROTEOME RESEARCH., vol. 10, no. 11, 4 November 2011 (2011-11-04), pages 5084-5094, XP055428905, US ISSN: 1535-3893, DOI: 10.1021/pr200498v
- EMILY S. BOJA ET AL: "Mass spectrometry-based targeted quantitative proteomics: Achieving sensitive and reproducible detection of proteins", PROTEOMICS, vol. 12, no. 8, 1 April 2012 (2012-04-01), pages 1093-1110, XP055429207, DE ISSN: 1615-9853, DOI: 10.1002/pmic.201100387
- WESLEY O. GREAVES ET AL: "Frequency and Spectrum of BRAF Mutations in a Retrospective, Single-Institution Study of 1112 Cases of Melanoma", JOURNAL OF MOLECULAR DIAGNOSTICS,THE, vol. 15, no. 2, 1 March 2013 (2013-03-01), pages 220-226, XP055428890, US ISSN: 1525-1578, DOI: 10.1016/j.jmoldx.2012.10.002
- GIDEON BOLLAG ET AL: "Vemurafenib: the first drug approved for BRAF-mutant cancer", NATURE REVIEWS. DRUG DISCOVERY, vol. 11, no. 11, 12 October 2012 (2012-10-12), pages 873-886, XP055273283, GB ISSN: 1474-1776, DOI: 10.1038/nrd3847
- Anonymous: "Quantitative Assays for RAS Pathway Proteins and Phosphorylation States", , 1 December 2014 (2014-12-01), pages 1-12, XP055429416, Retrieved from the Internet: URL:https://www.cancer.gov/research/key-in itiatives/ras/ras-central/blog/2014/ras-qu antitative-assays [retrieved on 2017-11-28]
- CATENACCI ET AL.: 'Absolute Quantitation of Met Using Mass Spectrometry for Clinical Application: Assay Precision, Stability, and Correlation with MET Gene Amplification in FFPE Tumor Tissue' PLOS ONE vol. 9, no. LSS. 7, 01 July 2014, pages 1 - 14, XP055163884

## Description

### Introduction

Specific peptides derived from subsequences of the Serine/Threonine-Protein Kinase B-raf, (also referred to as Proto-Oncogene B-Raf, p94, v-Raf murine sarcoma viral oncogene homolog B1, and BRAF, and referred to herein as "BRAF") are provided. Methods for the detection of BRAF levels in biological samples are known and disclosed, for example, in Dillon et al. (Dillon, R. et al. (2011) J. Proteome Res. 10: 5084-5094) as well as in patent publication WO 2014/037977. The peptide sequence and fragmentation/transition ions for each peptide provided herein are useful in a mass spectrometry-based Selected Reaction Monitoring (SRM) assay, which can also be referred to as a Multiple Reaction Monitoring (MRM) assay, and referred to herein as SRM/MRM. The use of peptides for SRM/MRM quantitative analysis of the BRAF protein is described.

This SRM/MRM assay as specified in claim 1 can be used to measure *relative* or *absolute* quantitative levels of the peptide of SEQ ID NO: 4 from the BRAF protein and therefore provides a method of measuring the amount of the BRAF protein in a given protein preparation obtained from a biological sample of formalin-fixed tissue by mass spectrometry.

More specifically, the SRM/MRM assay can measure these peptides directly in complex protein lysate samples prepared from cells procured from cancer patient tissue samples of formalin-fixed tissue. Methods of preparing protein samples from formalin-fixed tissue are described in U.S. Patent No. 7,473,532. The methods described in U.S. Patent No. 7,473,532 may conveniently be carried out using Liquid Tissue® reagents and protocol available from Expression Pathology Inc. (Rockville, MD).

The most widely and advantageously available form of tissues from cancer patients' tissue is formalin-fixed, paraffin embedded tissue. Formaldehyde/formalin-fixation of surgically removed tissue is by far the most common method of preserving cancer tissue samples worldwide and is the accepted convention for standard pathology practice. Aqueous solutions of formaldehyde are referred to as formalin. "100%" formalin consists of a saturated solution of formaldehyde (about 40% by volume or 37% by mass) in water, with a small amount of stabilizer, usually methanol to limit oxidation and degree of polymerization. The most common way in which tissue is preserved is to soak whole tissue for extended periods of time (8 hours to 48 hours) in aqueous formaldehyde, commonly termed 10% neutral buffered formalin, followed by embedding the fixed whole tissue in paraffin wax for long term storage at room temperature. Thus, molecular analytical methods to analyze formalin-fixed cancer tissue will be the most accepted and heavily utilized methods for analysis of cancer patient tissue.

Results from the SRM/MRM assay as specified in claim 1 can be used to correlate accurate and precise quantitative levels of the BRAF protein within the specific tissue samples (e.g., cancer tissue sample) of the patient or subject from whom the tissue (biological sample) was collected and preserved. This not only provides diagnostic and prognostic information about the cancer, but also allows a physician or other medical professional to more accurately determine appropriate therapy for the patient. Such an assay that provides diagnostically, prognostically, and therapeutically important information about levels of protein expression in a diseased tissue or other patient sample is termed a *companion diagnostic* assay. For example, such an assay can be designed to diagnose the stage or degree of a cancer and determine a therapeutic agent to which a patient is most likely to respond.

### Summary

The method as specified in claim 1 measures *relative* or *absolute* levels of the BRAF fragment peptide of SEQ ID NO:4. The peptide may be modified or unmodified. Examples of modifications include phosphorylated amino acid residues and glycosylated amino acid residues that may be present on the peptides.

*Relative* quantitative levels of the BRAF protein are determined by the SRM/MRM methodology by, for example, comparing SRM/MRM signature peak areas (e.g., signature peak area or integrated fragment ion intensity) of the BRAF fragment peptide of SEQ ID NO:4 in different samples. Alternatively, it may also be possible to further compare multiple SRM/MRM signature peak areas for multiple other BRAF signature peptides, where each peptide has its own specific SRM/MRM signature peak, to determine the relative BRAF protein content in one biological sample and compare it with the BRAF protein content in one or more additional or different biological samples. In this way, the amount of a particular peptide, or peptides, from the BRAF protein, and therefore the amount of the BRAF protein, is determined relative to the same BRAF peptide, or peptides, across 2 or more biological samples under the same experimental conditions. In addition, relative quantitation can also be determined for the BRAF fragment peptide of SEQ ID NO:4, and for further peptides, if applicable, from the BRAF protein within a single sample by comparing the signature peak area for that peptide by SRM/MRM methodology to the signature peak area for another and different peptide, or peptides, from a different protein, or proteins, within the same protein preparation from the biological sample. In this way, the amount of a particular peptide from the BRAF protein, and therefore the amount of the BRAF protein, is determined relative one to another within the same sample. These approaches permit quantitation of an individual peptide, or peptides, from the BRAF protein to the amount of another peptide, or peptides, between samples and within samples wherein the amounts as determined by signature peak area are relative one to another, regardless of the absolute weight to volume or weight to weight amounts of the BRAF peptide in the protein preparation from the biological sample. Relative quantitative data about individual signature peak areas between different samples can be normalized to the amount of protein analyzed per sample. Relative quantitation can be performed across many peptides from multiple proteins and the BRAF protein simultaneously in a single sample and/or across many samples to gain insight into relative protein amounts of one peptide/protein with respect to other peptides/proteins.

*Absolute* quantitative levels of the BRAF protein are determined by, for example, the SRM/MRM methodology whereby the SRM/MRM signature peak area of the BRAF fragment peptide of SEQ ID NO:4 in one biological sample is compared to the SRM/MRM signature peak area of a spiked internal standard. In one embodiment, the internal standard is a synthetic version of the same exact BRAF peptide that contains one or more amino acid residues labeled with one or more heavy isotopes. Such an isotope labeled internal standard is synthesized so that, when analyzed by mass spectrometry, it generates a predictable and consistent SRM/MRM signature peak that is different and distinct from the native BRAF peptide signature peak and which therefore can be used as a comparator peak. Thus, when the internal standard is spiked into a protein preparation from a biological sample in known amounts and analyzed by mass spectrometry, the SRM/MRM signature peak area of the native peptide is compared to the SRM/MRM signature peak area of the internal standard peptide, and this numerical comparison indicates either the absolute molarity and/or absolute weight of the native peptide present in the original protein preparation from the biological sample. Absolute quantitative data for fragment peptides are displayed according to the amount of protein analyzed per sample. Absolute quantitation can be performed across many peptides, and thus proteins, simultaneously in a single sample and/or across many samples to gain insight into absolute protein amounts in individual biological samples and in entire cohorts of individual samples.

The SRM/MRM assay method described herein can be used to aid diagnosis of the stage of a cancer and/or the patient prognosis directly in patient-derived, formalin-fixed tissue, and to aid in determining which therapeutic agent would be most advantageous for use in treating that patient. Cancer tissue that is removed from a patient either through surgery, such as for therapeutic removal of partial or entire tumors, or through biopsy procedures conducted to determine the presence or absence of suspected disease, is analyzed to determine whether or not a specific protein, or proteins, and which forms of proteins, are present in that patient tissue. Moreover, the expression level of a protein, or multiple proteins, can be determined and compared to a "normal" or reference level found in healthy tissue. Normal or reference levels of proteins found in healthy tissue may be derived from, for example, the relevant tissues of one or more individuals that do not have cancer. Alternatively, normal or reference levels may be obtained for individuals with cancer by analysis of relevant tissues not affected by the cancer.

Assays of protein levels (e.g., BRAF levels) can also be used to diagnose the stage of cancer and provide prognostic information about a patient or subject diagnosed with cancer by employing the BRAF levels. The level of an individual BRAF peptide is defined as the molar amount of the peptide determined by the SRM/MRM assay per total amount of protein lysate analyzed. Information regarding BRAF can thus be used to aid in determining the stage or grade of a cancer and/or patient prognosis by correlating the level of the BRAF protein (or fragment peptides of the BRAF protein) with levels observed in normal tissues. Once the stage and/or grade, and/or BRAF protein expression characteristics of the cancer has been determined, that information can be matched to a list of therapeutic agents (chemical and biological) developed to specifically treat cancer tissue that is characterized by, for example, abnormal expression of the protein or protein(s) (e.g., BRAF) that were assayed. The therapeutic agents Vemurafenib and Sorafenib are particularly useful for targeting BRAF-expressing cancer cells. Other BRAF inhibitors include GDC-0879, PLX-4720, dabrafenib, and LGX818. Matching information from a BRAF protein assay to a list of therapeutic agents that specifically targets, for example, the BRAF protein or cells/tissue expressing the protein, defines what has been termed a *personalized medicine* approach to treating disease. The assay methods described herein form the foundation of a *personalized medicine* approach by using analysis of proteins from the patient's own tissue as a source for diagnostic and treatment decisions.

### Brief Description of the Drawings

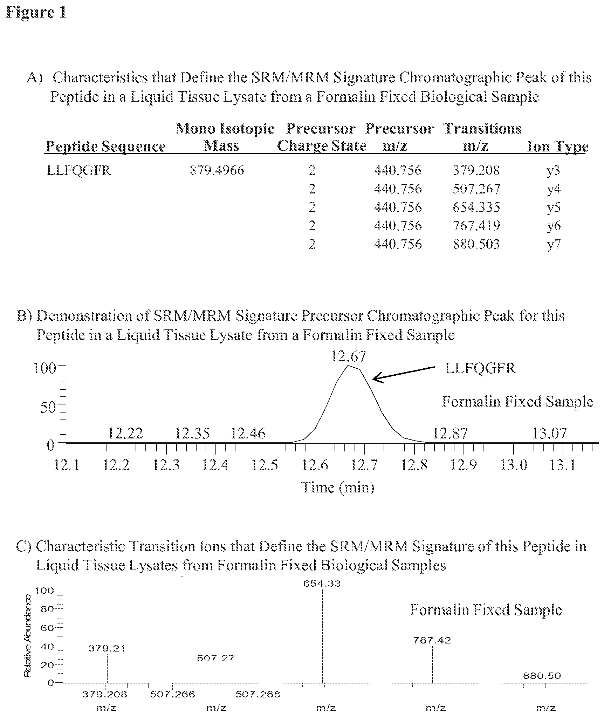

Figure 1, parts A to C, shows an example of an SRM/MRM assay of the BRAF fragment peptide of SEQ ID NO:4 performed on a Liquid Tissue lysate from a formalin-fixed biological sample with quantitation of the BRAF peptide conducted on a triple quadrupole mass spectrometer. The specific characteristics about how to measure this peptide in biological samples that have been fixed in formalin are shown.

### Detailed Description

The BRAF fragment peptide of SEQ ID NO:4 is used as a surrogate reporter to determine the abundance of BRAF protein in a sample using a mass spectrometry-based SRM/MRM assay.

BRAF fragment peptides may be generated by a variety of methods including by the use of the Liquid Tissue protocol provided in US Patent 7,473,532. The Liquid Tissue protocol and reagents are capable of producing peptide samples suitable for mass spectroscopic analysis from formalin-fixed paraffin-embedded tissue by proteolytic digestion of the proteins in the tissue/biological sample. In the Liquid Tissue protocol the tissue/biological is heated in a buffer for an extended period of time (e.g., from about 80° C to about 100° C for a period of time from about 10 minutes to about 4 hours) to reverse or release protein cross-linking. The buffer employed is a neutral buffer, (e.g., a Tris-based buffer, or a buffer containing a detergent). Following heat treatment, the tissue/biological sample is treated with one or more proteases, including but not limited to trypsin, chymotrypsin, pepsin, and endo-proteinase Lys-C, for a time sufficient to disrupt the tissue and cellular structure of said biological sample and to liquefy the sample (e.g., a period of time from 30 minutes to 24 hours at a temperature from 37° C to 65° C). The result of the heating and proteolysis is a liquid, soluble, dilutable biomolecule lysate.

Surprisingly, it was found that many potential peptide sequences from the BRAF protein are unsuitable or ineffective for use in mass spectrometry-based SRM/MRM assays for reasons that are not immediately evident. This is particularly true for peptides derived from formalin-fixed tissue. As it was not possible to predict the most suitable peptides for MRM/SRM assay, it was necessary to experimentally identify suitable peptides in actual Liquid Tissue lysates to develop a reliable and accurate SRM/MRM assay for the BRAF protein. While not wishing to be bound by any theory, it is believed that some peptides might, for example, be difficult to detect by mass spectrometry as they do not ionize well or produce fragments that are not distinct from other proteins. Peptides may also fail to resolve well in separation (e.g., liquid chromatography), or may adhere to glass or plastic ware.

The BRAF peptide of SEQ ID NO:4 and further BRAF peptides identified (e.g., Tables 1 and 2) were derived from the BRAF protein by protease digestion of all the proteins within a complex Liquid Tissue lysate prepared from cells procured from formalin-fixed cancer tissue. Unless noted otherwise, in each instance the protease was trypsin. The Liquid Tissue lysate was then analyzed by mass spectrometry to determine those peptides derived from the BRAF protein that are detected and analyzed by mass spectrometry. Identification of a specific preferred subset of peptides for mass-spectrometric analysis is based on; 1) experimental determination of which peptide or peptides from a protein ionize in mass spectrometry analyses of Liquid Tissue lysates, and 2) the ability of the peptide to survive the protocol and experimental conditions used in preparing a Liquid Tissue lysate. This latter property extends not only to the amino acid sequence of the peptide but also to the ability of a modified amino acid residue within a peptide to survive in modified form during the sample preparation.

Protein lysates from cells procured directly from formalin (formaldehyde) fixed tissue were prepared using the Liquid Tissue reagents and protocol that entails collecting cells into a sample tube via tissue microdissection followed by heating the cells in the Liquid Tissue buffer for an extended period of time. Once the formalin-induced cross linking has been negatively affected, the tissue/cells are then digested to completion in a predictable manner using a protease, such as, for example, trypsin (although other proteases can be used). Each protein lysate is turned into a collection of peptides by digestion of intact polypeptides with the protease. Each Liquid Tissue lysate was analyzed (e.g., by ion trap mass spectrometry) to perform multiple global proteomic surveys of the peptides, where the data was presented as identification of as many peptides as could be identified by mass spectrometry from all cellular proteins present in each protein lysate. An ion trap mass spectrometer or another form of a mass spectrometer that is capable of performing global profiling for identification of as many peptides as possible from a single complex protein/peptide lysate is employed. Ion trap mass spectrometers however may advantageously be used conducting global profiling of peptides. Although an SRM/MRM assay can be developed and performed on any type of mass spectrometer, including a MALDI, ion trap, or triple quadrupole, advantageously a triple quadrupole instrument platform is used for an SRM/MRM assay. That type of a mass spectrometer is suitable instrument for analyzing a single isolated target peptide within a very complex protein lysate that may consist of hundreds of thousands to millions of individual peptides from all the proteins contained within a cell.

Once as many peptides as possible were identified in a single MS analysis of a single lysate under the conditions employed, then that list of peptides was collated and used to determine the proteins that were detected in that lysate. That process was repeated for multiple Liquid Tissue lysates, and the very large list of peptides was collated into a single dataset. That type of dataset can be considered to represent the peptides that can be detected in the type of biological sample that was analyzed (after protease digestion), and specifically in a Liquid Tissue lysate of the biological sample, and thus includes the peptides for specific proteins, such as for example the BRAF protein.

The BRAF tryptic peptide identified as useful in the determination of absolute or relative amounts of the BRAF protein was the peptide of SEQ ID NO:4. Further suitable peptides are listed in Table 1. Each of those peptides was detected by mass spectrometry in Liquid Tissue lysates prepared from formalin-fixed, paraffin embedded tissue.

**Table 1**

| **SEQ ID** | **Peptide sequence** |
|---|---|
| SEQ ID NO: 1 | LTQEHIEALLDK |
| SEQ ID NO: 2 | TWPAR |
| SEQ ID NO: 3 | IQDGEK |
| SEQ ID NO: 4 | LLFQGFR |
| SEQ ID NO: 5 | NQFGQR |
| SEQ ID NO: 6 | NEVGVLR |
| SEQ ID NO: 7 | SNNIFLHEDLTVK |
| SEQ ID NO: 8 | SASEPSLNR |
| SEQ ID NO: 9 | FGGEHNPPSIYLEAYEEYTSK |
| SEQ ID NO: 10 | SSSSSEDR |
| SEQ ID NO: 11 | TPIQAGGYGAFPVH |

The BRAF tryptic peptides listed in Table 1 were detected from multiple Liquid Tissue lysates of multiple different formalin-fixed tissues of different human organs including prostate, colon, and breast. Each of those peptides other than that of SEQ ID NO:4 is also considered useful for quantitative SRM/MRM assay of the BRAF protein in formalin-fixed tissue. Further data analysis of these experiments indicated no preference for any specific peptides from any specific organ site. Thus, these peptides other than that of SEQ ID NO:4 may also be used for conducting SRM/MRM assays of the BRAF protein on a Liquid Tissue lysate from any formalin-fixed tissue originating from any biological sample or from any organ site in the body.

In order to most efficiently implement an SRM/MRM assay for the BRAF peptide of SEQ ID NO:4 it is desirable to utilize information in addition to the peptide sequence in the analysis. That additional information may be used in directing and instructing the mass spectrometer (e.g. a triple quadrupole mass spectrometer) to perform the correct and focused analysis of specific targeted peptide(s), such that the assay may be effectively performed.

The additional information about target peptides in general, and about specific BRAF peptides, may include one or more of the mono isotopic mass of the peptide, its precursor charge state, the precursor m/z value, the m/z transition ions, and the ion type of each transition ion. Table 2 shows additional peptide information that may be used to develop an SRM/MRM assay for the BRAF protein for two (2) of the BRAF peptides from the list in Table 1. Similar additional information described for the two (2) BRAF peptides shown by example in Table 2 may be prepared, obtained, and applied to the analysis of the other peptides contained in Table 1.

**Table 2**

| **SEQ ID** | **Peptide sequence** | **Mono Isotopic Mass** | **Precursor Charge State** | **Precursor m/z** | **Transition m/z** | **Ion Type** |
|---|---|---|---|---|---|---|
| SEQ ID NO: 1 | LTQEHIEALLDK | 1408.7562 | 2 | 705.385 | 688.387 | y6 |
| | | | 2 | 705.385 | 801.471 | y7 |
| | | | 2 | 705.385 | 938.53 | y8 |
| | | | 2 | 705.385 | 1067.573 | y9 |
| | | | 2 | 705.385 | 1195.631 | y10 |
| SEQ ID NO: 4 | LLFQGFR | 879.4966 | 2 | 440.756 | 379.208 | y3 |
| | | | 2 | 440.756 | 507.267 | y4 |
| | | | 2 | 440.756 | 654.335 | y5 |
| | | | 2 | 440.756 | 767.419 | y6 |
| | | | 2 | 440.756 | 880.503 | y7 |

The method described below was used to: 1) identify candidate peptides from the BRAF protein that can be used for a mass spectrometry-based SRM/MRM assay for the BRAF protein, 2) develop an individual SRM/MRM assay, or assays, for target peptides from the BRAF protein in order to correlate and 3) apply quantitative assays to cancer diagnosis and/or choice of optimal therapy.

### Assay Method

1. Identification of SRM/MRM candidate fragment peptides for the BRAF protein
   a. Prepare a Liquid Tissue protein lysate from a formalin fixed biological sample using a protease or proteases, (that may or may not include trypsin), to digest proteins
   b. Analyze all protein fragments in the Liquid Tissue lysate on an ion trap tandem mass spectrometer and identify all fragment peptides from the BRAF protein, where individual fragment peptides do not contain any peptide modifications such as phosphorylations or glycosylations
   c. Analyze all protein fragments in the Liquid Tissue lysate on an ion trap tandem mass spectrometer and identify all fragment peptides from the BRAF protein that carry peptide modifications such as for example phosphorylated or glycosylated residues
   d. All peptides generated by a specific digestion method from the entire, full length BRAF protein potentially can be measured, but preferred peptides used for development of the SRM/MRM assay are those that are identified by mass spectrometry directly in a complex Liquid Tissue protein lysate prepared from a formalin fixed biological sample
   e. Peptides that are specifically modified (phosphorylated, glycosylated, etc.) in patient tissue and which ionize, and thus detected, in a mass spectrometer when analyzing a Liquid Tissue lysate from a formalin fixed biological sample are identified as candidate peptides for assaying peptide modifications of the BRAF protein
2. Mass Spectrometry Assay for Fragment Peptides from the BRAF Protein
   a. SRM/MRM assay on a triple quadrupole mass spectrometer for individual fragment peptides identified in a Liquid Tissue lysate is applied to peptides from the BRAF protein
      i. Determine optimal retention time for a fragment peptide for optimal chromatography conditions including liquid chromatography, nano-reversed phase liquid chromatography, high performance liquid chromatography, and reverse phase high performance liquid chromatography
      ii. Determine the mono isotopic mass of the peptide, the precursor charge state for each peptide, the precursor m/z value for each peptide, the m/z transition ions for each peptide, and the ion type of each transition ion for each fragment peptide in order to develop an SRM/MRM assay for each peptide.
      iii. SRM/MRM assay can then be conducted using the information from (i) and (ii) on a triple quadrupole mass spectrometer where each peptide has a characteristic and unique SRM/MRM signature peak that precisely defines the unique SRM/MRM assay as performed on a triple quadrupole mass spectrometer
   b. Perform SRM/MRM analysis so that the amount of the fragment peptide of the BRAF protein that is detected, as a function of the unique SRM/MRM signature peak area from an SRM/MRM mass spectrometry analysis, can indicate both the relative and absolute amount of the protein in a particular protein lysate.
      i. Relative quantitation may be achieved by:
         1. Determining increased or decreased presence of the BRAF protein by comparing the SRM/MRM signature peak area from a given BRAF peptide detected in a Liquid Tissue lysate from one formalin fixed biological sample to the same SRM/MRM signature peak area of the same BRAF fragment peptide in at least a second, third, fourth or more Liquid Tissue lysates from least a second, third, fourth or more formalin fixed biological samples
         2. Determining increased or decreased presence of the BRAF protein by comparing the SRM/MRM signature peak area from a given BRAF peptide detected in a Liquid Tissue lysate from one formalin fixed biological sample to SRM/MRM signature peak areas developed from fragment peptides from other proteins, in other samples derived from different and separate biological sources, where the SRM/MRM signature peak area comparison between the 2 samples for a peptide fragment are normalized to amount of protein analyzed in each sample.
         3. Determining increased or decreased presence of the BRAF protein by comparing the SRM/MRM signature peak area for a given BRAF peptide to the SRM/MRM signature peak areas from other fragment peptides derived from different proteins within the same Liquid Tissue lysate from the formalin fixed biological sample in order to normalize changing levels of BRAF protein to levels of other proteins that do not change their levels of expression under various cellular conditions.
         4. These assays can be applied to both unmodified fragment peptides and for modified fragment peptides of the BRAF protein, where the modifications include but are not limited to phosphorylation and/or glycosylation, and where the relative levels of modified peptides are determined in the same manner as determining relative amounts of unmodified peptides.
      ii. Absolute quantitation of a given peptide may be achieved by comparing the SRM/MRM signature peak area for a given fragment peptide from the BRAF protein in an individual biological sample to the SRM/MRM signature peak area of an internal fragment peptide standard spiked into the protein lysate from the biological sample
         1. The internal standard is a labeled synthetic version of the fragment peptide from the BRAF protein that is being interrogated. This standard is spiked into a sample in known amounts, and the SRM/MRM signature peak area can be determined for both the internal fragment peptide standard and the native fragment peptide in the biological sample separately, followed by comparison of both peak areas
         2. This can be applied to unmodified fragment peptides and modified fragment peptides, where the modifications include but are not limited to phosphorylation and/or glycosylation, and where the absolute levels of modified peptides can be determined in the same manner as determining absolute levels of unmodified peptides.
3. Apply Fragment Peptide Quantitation to Cancer Diagnosis and Treatment
   a. Perform relative and/or absolute quantitation of fragment peptide levels of the BRAF protein and demonstrate that the previously-determined association, as well understood in the field of cancer, of BRAF protein expression to the stage/grade/status of cancer in patient tumor tissue is confirmed
   b. Perform relative and/or absolute quantitation of fragment peptide levels of the BRAF protein and demonstrate correlation with clinical outcomes from different treatment strategies, wherein this correlation has already been demonstrated in the field or can be demonstrated in the future through correlation studies across cohorts of patients and tissue from those patients. Once either previously established correlations or correlations derived in the future are confirmed by this assay then the assay method can be used to determine optimal treatment strategy

Figure 1 shows an example of a single SRM/MRM assay as specified in claim 1 performed on a Liquid Tissue lysate from a formalin-fixed biological sample. An SRM/MRM assay was developed for the peptide of SEQ ID NO:4 for quantitation of the BRAF protein on a triple quadrupole mass spectrometer. Specific and unique characteristics about this BRAF peptide of SEQ ID NO:4 (sequence LLFQGFR) were developed by analysis of all BRAF peptides on both an ion trap and triple quadrupole mass spectrometers and are shown in Figure 1A. That information includes the monoisotopic mass of the peptide, its precursor charge state, the precursor m/z value, the transition m/z values of the precursor, and the ion types of each of the identified transitions. That information must be determined experimentally for each and every candidate SRM/MRM peptide directly in Liquid Tissue lysates from formalin-fixed samples/tissue, because, interestingly, not all peptides from the BRAF protein can be detected in such lysates using SRM/MRM as described herein, indicating that BRAF peptides not detected cannot be considered candidate peptides for developing an SRM/MRM assay for use in quantitating peptides/proteins directly in Liquid Tissue lysates from formalin-fixed samples/tissue.

As shown in Figure 1B, this particular SRM/MRM assay was performed on a triple quadrupole mass spectrometer. The experimental sample in this experiment was a Liquid Tissue protein lysate prepared from a cell line that had been formalin-fixed, paraffin-embedded to act as a tissue surrogate. Data from the assay indicate the presence of the unique SRM/MRM signature peak for the BRAF peptide of SEQ ID NO:4 in the formalin-fixed sample.

Figure 1C shows the specific transition ion characteristics for this peptide used to quantitatively measure the above-mentioned peptide in formalin-fixed biological samples. These data indicate absolute amounts of this BRAF peptide as a function of the molar amount of the peptide per microgram of protein lysate analyzed. Assessment of BRAF protein levels in tissues based on analysis of formalin fixed patient-derived tissue can provide diagnostic, prognostic, and therapeutically-relevant information about each particular patient.

This disclosure describes a method for measuring the level of the Serine/Threonine-Protein Kinase B-raf (BRAF) protein in a biological sample of formalin-fixed tissue, comprising detecting and quantifying the amount of a BRAF fragment peptide in a protein digest prepared from said biological sample using mass spectrometry; and calculating the level of BRAF protein in said sample; wherein said BRAF fragment peptide is the peptide of SEQ ID NO:4; and wherein the level is a relative level or an absolute level. In one embodiment of the present invention, quantifying the BRAF fragment peptide comprises determining the amount of the BRAF fragment peptide of SEQ ID NO:4 in a biological sample by comparison to an added internal standard peptide of known amount, wherein the BRAF fragment peptide in the biological sample is compared to an internal standard peptide having the same amino acid sequence. In some embodiments the internal standard is an isotopically labeled internal standard peptide comprises one or more heavy stable isotopes selected from ¹⁸O, ¹⁷O, ³⁴S, ¹⁵N, ¹³C, ²H, and combinations thereof.

The method for measuring the level of the BRAF protein in a biological sample described herein may be used as a diagnostic and/or prognostic indicator of cancer in a patient or subject. In one embodiment, the results from measurements of the level of the BRAF protein may be employed to determine the diagnostic stage/grade/status and/or the prognostic status of a cancer by correlating (e.g., comparing) the level of BRAF protein found in a tissue with the level of that protein found in normal and/or cancerous or precancerous tissues.

Because both nucleic acids and protein can be analyzed from the same Liquid Tissue™ biomolecular preparation it is possible to generate additional information about disease diagnosis and drug treatment decisions from the nucleic acids in same sample upon which proteins were analyzed. For example, if the BRAF protein is expressed by certain cells at increased levels, when assayed by SRM the data can provide information about the state of the cells and their potential for uncontrolled growth, potential drug resistance and the development of cancers can be obtained. At the same time, information about the status of the BRAF genes and/or the nucleic acids and proteins they encode (e.g., mRNA molecules and their expression levels or splice variations) can be obtained from nucleic acids present in the same Liquid Tissue™ biomolecular preparation can be assessed simultaneously to the SRM analysis of the BRAF protein. Any gene and/or nucleic acid not from the BRAF and which is present in the same biomolecular preparation can be assessed simultaneously to the SRM analysis of the BRAF protein. In one instance, information about the BRAF protein and/or one, two, three, four or more additional proteins may be assessed by examining the nucleic acids encoding those proteins. Those nucleic acids can be examined, for example, by one or more, two or more, or three or more of: sequencing methods, polymerase chain reaction methods, restriction fragment polymorphism analysis, identification of deletions, insertions, and/or determinations of the presence of mutations, including but not limited to, single base pair polymorphisms, transitions, transversions, or combinations thereof.

## Claims

1. A method for measuring the level of the BRAF tyrosine kinase receptor protein (BRAF) in a human biological sample of formalin-fixed tissue, comprising detecting and quantifying the amount of a BRAF fragment peptide in a protein digest prepared from said biological sample using mass spectrometry; and calculating the level of BRAF protein in said sample; wherein said BRAF fragment peptide is the peptide of SEQ ID NO:4; and wherein said level is a relative level or an absolute level.

2. The method of claim 1, further comprising the step of fractionating said protein digest prior to detecting and quantifying the amount of said BRAF fragment peptide, wherein said fractionating step is selected from the group consisting of liquid chromatography, nano-reversed phase liquid chromatography, high performance liquid chromatography, or reverse phase high performance liquid chromatography.

3. The method of claim 1 or 2, wherein said protein digest of said biological sample comprises a protease digest.

4. The method of any of claims 1 to 3, wherein the mode of mass spectrometry used is Selected Reaction Monitoring (SRM), Multiple Reaction Monitoring (MRM), and/or multiple Selected Reaction Monitoring (mSRM).

5. The method of any of claims 1 to 4, wherein the tissue is paraffin embedded tissue.

6. The method of claim 5, wherein the tissue is obtained from a tumor.

7. The method of any of claims 1 to 6, wherein quantifying the BRAF fragment peptide comprises comparing an amount of said BRAF fragment peptide in one biological sample to the amount of the same BRAF fragment peptide in a different and separate biological sample.

8. The method of claim 7, wherein quantifying said BRAF fragment peptide comprises determining the amount of said BRAF fragment peptide in a biological sample by comparison to an added internal standard peptide of known amount, wherein said BRAF fragment peptide in the biological sample is compared to an internal standard peptide having the same amino acid sequence.

9. The method of claim 8, wherein the internal standard peptide is an isotopically labeled peptide comprising one or more heavy stable isotopes selected from ¹⁸O, ¹⁷O, ³⁴S, ¹⁵N, ¹³C, ²H and combinations thereof.

10. The method of any of claims 1 to 9, wherein detecting and quantifying the amount of said BRAF fragment peptide in the protein digest indicates the presence of BRAF protein and an association with cancer in the subject from whom the biological sample was obtained.

11. The method of claim 10, further comprising correlating the results of said detecting and quantifying the amount of said BRAF fragment peptide, or the level of said BRAF protein to the diagnostic stage/grade/status of the cancer.

12. The method of claim 11, wherein correlating the results of said detecting and quantifying the amount of said BRAF fragment peptide, or the level of said BRAF protein to the diagnostic stage/grade/status of the cancer is combined with detecting and/or quantifying the amount of other proteins or peptides from other proteins in a multiplex format to provide additional information about the diagnostic stage/grade/status of the cancer.

13. The method of any one of claims 1 to 12, further comprising selecting for the subject from which said biological sample was obtained a therapeutic agent based on the presence, absence, or amount of said BRAF fragment peptide or the level of BRAF protein.

14. The method of claim 13, wherein therapeutic agent binds the BRAF protein and/or inhibits its biological activity.

15. The method of claim 14, wherein the therapeutic agent is selected from Vemurafenib or Sorafenib.

## Patentansprüche

1. Verfahren zum Messen der Konzentration des BRAF-Tyrosinkinaserezeptorproteins (BRAF) in einer humanen biologischen Probe von formalinfixiertem Gewebe, bei dem man die Menge eines BRAF-Fragmentpeptids in einem von der biologischen Probe hergestellten Proteinverdau unter Anwendung von Massenspektrometrie nachweist und quantifiziert und die Konzentration des BRAF-Proteins in der Probe berechnet, wobei es sich bei dem BRAF-Fragmentpeptid um das Peptid der SEQ ID NO: 4 handelt und wobei es sich bei der Konzentration um eine relative Konzentration oder eine absolute Konzentration handelt.

2. Verfahren nach Anspruch 1, weiterhin umfassend den Schritt der Fraktionierung des Proteinverdaus vor dem Nachweis und der Quantifizierung der Menge des BRAF-Fragmentpeptids, wobei der Fraktionierungsschritt aus der aus Flüssigchromatographie, Nanoumkehrphasenflüssigchromatografie, Hochleistungsflüssigchromatografie oder Umkehrphasenhochleistungsflüssigchromatografie bestehenden Gruppe ausgewählt ist.

3. Verfahren nach Anspruch 1 oder 2, wobei der Proteinverdau der biologischen Probe einen Proteaseverdau umfasst.

4. Verfahren nach einem der Ansprüche 1 bis 3, wobei es sich bei dem angewendeten Massenspektrometriemodus um Selected Reaction Monitoring (SRM), Multiple Reaction Monitoring (MRM) und/oder Multiple Selected Reaction Monitoring (mSRM) handelt.

5. Verfahren nach einem der Ansprüche 1 bis 4, wobei es sich bei dem Gewebe um in Paraffin eingebettetes Gewebe handelt.

6. Verfahren nach Anspruch 5, wobei das Gewebe von einem Tumor erhalten wird.

7. Verfahren nach einem der Ansprüche 1 bis 6, wobei die Quantifizierung des BRAF-Fragmentpeptids den Vergleich einer Menge des BRAF-Fragmentpeptids in einer biologischen Probe mit der Menge des gleichen BRAF-Fragmentpeptids in einer verschiedenen und separaten biologischen Probe umfasst.

8. Verfahren nach Anspruch 7, wobei das Quantifizieren des BRAF-Fragmentpeptids die Bestimmung der Menge des BRAF-Fragmentpeptids in einer biologischen Probe durch Vergleich mit einem zugefügten Interner-Standard-Peptid einer bekannten Menge umfasst, wobei man das BRAF-Fragmentpeptid in der biologischen Probe mit einem Interner-Standard-Peptid mit der gleichen Aminosäuresequenz vergleicht.

9. Verfahren nach Anspruch 8, wobei es sich bei dem Interner-Standard-Peptid um ein isotopenmarkiertes Peptid handelt, das einen oder mehrere schwere stabile Isotope ausgewählt aus ¹⁸O, ¹⁷O, ³⁴S, ¹⁵N, ¹³C, ²H und Kombinationen davon umfasst.

10. Verfahren nach einem der Ansprüche 1 bis 9, wobei der Nachweis und die Quantifizierung der Menge des BRAF-Fragmentpeptids in dem Peptidverdau das Vorhandensein von BRAF-Protein und einer Assoziation mit Krebs in dem Subjekt, von dem die biologische Probe erhalten wurde, anzeigt.

11. Verfahren nach Anspruch 10, weiterhin umfassend das Korrelieren der Ergebnisse des Nachweises und der Quantifizierung der Menge des BRAF-Fragmentpeptids oder der Konzentration des BRAF-Proteins mit dem diagnostischen Stadium/Grad/Status der Krebserkrankung.

12. Verfahren nach Anspruch 11, wobei man das Korrelieren der Ergebnisse des Nachweises und der Quantifizierung der Menge des BRAF-Fragmentpeptids oder der Konzentration des BRAF-Proteins mit dem diagnostischen Stadium/Grad/Status der Krebserkrankung mit dem Nachweis und/oder der Quantifizierung der Menge anderer Proteine oder Peptide von anderen Proteinen in einem Multiplexformat kombiniert, unter Bereitstellung zusätzlicher Informationen über das diagnostische Stadium/den diagnostischen Grad/den diagnostischen Status der Krebserkrankung.

13. Verfahren nach einem der Ansprüche 1 bis 12, bei dem man weiterhin für das Subjekt, von dem die biologische Probe erhalten wurde, basierend auf dem Vorhandensein, der Abwesenheit oder der Menge des BRAF-Fragmentpeptids oder der Konzentration an BRAF-Protein ein Therapeutikum auswählt.

14. Verfahren nach Anspruch 13, wobei das Therapeutikum an das BRAF-Protein bindet und/oder dessen biologische Aktivität hemmt.

15. Verfahren nach Anspruch 14, wobei das Therapeutikum aus Vemurafenib und Sorafenib ausgewählt ist.

## Revendications

1. Procédé pour la mesure du niveau de la protéine du récepteur à activité tyrosine kinase BRAF (BRAF) dans un échantillon biologique humain de tissu fixé au formol, comprenant la détection et la quantification de la quantité d'un fragment peptidique de BRAF dans une digestion de protéines préparée à partir dudit échantillon biologique à l'aide de spectrométrie de masse ; et le calcul du niveau de protéine BRAF dans ledit échantillon ; ledit fragment peptidique de BRAF étant le peptide de SEQ ID NO : 4 ; et ledit niveau étant un niveau relatif ou un niveau absolu.

2. Procédé selon la revendication 1, comprenant en outre l'étape de fractionnement de ladite digestion de protéines avant la détection et la quantification de la quantité dudit fragment peptidique de BRAF, ladite étape de fractionnement étant choisie dans le groupe constitué par la chromatographie liquide, la nano-chromatographie liquide en phase inverse, la chromatographie liquide à haute performance, ou la chromatographie liquide à haute performance en phase inverse.

3. Procédé selon la revendication 1 ou 2, ladite digestion de protéines dudit échantillon biologique comprenant une digestion de protéases.

4. Procédé selon l'une quelconque des revendications 1 à 3, le mode de spectrométrie de masse utilisé étant le suivi de réaction sélectionnée (SRS), le suivi de réactions multiples (SRM), et/ou le suivi de réactions sélectionnées multiples (SRSm).

5. Procédé selon l'une quelconque des revendications 1 à 4, le tissu étant un tissu enrobé de paraffine.

6. Procédé selon la revendication 5, le tissu étant obtenu à partir d'une tumeur.

7. Procédé selon l'une quelconque des revendications 1 à 6, la quantification du fragment peptidique de BRAF comprenant la comparaison d'une quantité dudit fragment peptidique de BRAF dans un échantillon biologique avec la quantité du même fragment peptidique de BRAF dans un échantillon biologique différent et distinct.

8. Procédé selon la revendication 7, la quantification dudit fragment peptidique de BRAF comprenant la détermination de la quantité dudit fragment peptidique de BRAF dans un échantillon biologique par comparaison avec un peptide standard interne ajouté de quantité connue, ledit fragment peptidique de BRAF dans l'échantillon biologique étant comparé à un peptide standard interne possédant la même séquence d'acides aminés.

9. Procédé selon la revendication 8, le peptide standard interne étant un peptide isotopiquement marqué comprenant un ou plusieurs isotopes stables lourds choisis parmi ¹⁸O, ¹⁷O, ³⁴S, ¹⁵N, ¹³C, ²H et des combinaisons correspondantes.

10. Procédé selon l'une quelconque des revendications 1 à 9, la détection et la quantification de la quantité dudit fragment peptidique de BRAF dans la digestion de protéines indiquant la présence de protéine BRAF et une association avec un cancer chez le sujet duquel l'échantillon biologique a été obtenu.

11. Procédé selon la revendication 10, comprenant en outre la corrélation des résultats de ladite détection et de ladite quantification de la quantité dudit fragment peptidique de BRAF, ou du niveau de ladite protéine BRAF avec le diagnostic stade/degré/statut du cancer.

12. Procédé selon la revendication 11, la corrélation des résultats de ladite détection et de ladite quantification de la quantité dudit fragment peptidique de BRAF, ou du niveau de ladite protéine BRAF avec le diagnostic stade/degré/statut du cancer étant combinée avec la détection et/ou la quantification de la quantité d'autres protéines ou de peptides d'autres protéines dans un format multiplex pour fournir des informations supplémentaires sur le diagnostic stade/degré/statut du cancer.

13. Procédé selon l'une quelconque des revendications 1 à 12, comprenant en outre la sélection, pour le sujet duquel ledit échantillon biologique a été obtenu, d'un agent thérapeutique, basé sur la présence, l'absence, ou la quantité dudit fragment peptidique de BRAF ou sur le niveau de ladite protéine BRAF.

14. Procédé selon la revendication 13, un agent thérapeutique se liant à la protéine BRAF et/ou inhibant son activité biologique.

15. Procédé selon la revendication 14, l'agent thérapeutique étant choisi parmi le Vémurafénib et le Sorafénib.


### SEQUENCE LISTING

<110> Expression Pathology Inc
<120> SRM/MRM Assay for the Serine/Threonine-Protein Kinase B-raf (BRAF)
<130> 001152-8038.WO00
<150> 62/023,615
   <151> 2014-07-11
<160> 11
<170> PatentIn version 3.5
<210> 1
   <211> 12
   <212> PRT
   <213> Homo sapiens
<400> 1
   Leu Thr Gln Glu His Ile Glu Ala Leu Leu Asp Lys
   1               5                   10
<210> 2
   <211> 6
   <212> PRT
   <213> Homo sapiens
<400> 2
   Thr Val Val Pro Ala Arg
   1               5
<210> 3
   <211> 6
   <212> PRT
   <213> Homo sapiens
<400> 3
   Ile Gln Asp Gly Glu Lys
   1               5
<210> 4
   <211> 7
   <212> PRT
   <213> Homo sapiens
<400> 4
   Leu Leu Phe Gln Gly Phe Arg
   1               5
<210> 5
   <211> 6
   <212> PRT
   <213> Homo sapiens
<400> 5
   Asn Gln Phe Gly Gln Arg
   1               5
<210> 6
   <211> 7
   <212> PRT
   <213> Homo sapiens
<400> 6
   Asn Glu Val Gly Val Leu Arg
   1               5
<210> 7
   <211> 13
   <212> PRT
   <213> Homo sapiens
<400> 7
   Ser Asn Asn Ile Phe Leu His Glu Asp Leu Thr Val Lys
   1               5                   10
<210> 8
   <211> 9
   <212> PRT
   <213> Homo sapiens
<400> 8
   Ser Ala Ser Glu Pro Ser Leu Asn Arg
   1               5
<210> 9
   <211> 21
   <212> PRT
   <213> Homo sapiens
<400> 9
   Phe Gly Gly Glu His Asn Pro Pro Ser Ile Tyr Leu Glu Ala Tyr Glu
   1               5                   10                  15
   Glu Tyr Thr Ser Lys
               20
<210> 10
   <211> 8
   <212> PRT
   <213> Homo sapiens
<400> 10
   Ser Ser Ser Ser Ser Glu Asp Arg
          1               5
<210> 11
   <211> 14
   <212> PRT
   <213> Homo sapiens
<400> 11
   Thr Pro Ile Gln Ala Gly Gly Tyr Gly Ala Phe Pro Val His
   1               5                   10